# EUROPEAN PATENT APPLICATION

(11) **EP 4 491 621 A1**
(43) Date of publication of application: **15.01.2025**
(21) Application number: 23766767.0
(22) Date of filing: 06.03.2023
(51) Int. Cl.: C07D 331/02

(54) **PRODUCTION METHOD FOR EPISULFIDE COMPOUND**

(30) Priority: 08.03.2022 JP 2022035005
(71) Applicant: MITSUBISHI GAS CHEMICAL COMPANY, INC., Chiyoda-ku Tokyo 100-8324 (JP)
(72) Inventor: SUGIHARA Ryosuke, Tokyo 125-8601 (JP); TAKEMURA Kouhei, Tokyo 125-8601 (JP); IMAGAWA Yousuke, Osaka-shi, Osaka 551-0022 (JP)
(74) Representative: Müller-Boré & Partner Patentanwälte PartG mbB
(86) International application number: PCT/JP2023/008215
(87) International publication number: WO 2023/171591

(57) **Abstract**

The present invention enables the provision of a production method for an episulfide compound, which comprises the steps of: (A) producing an episulfide compound having two or more units of the structure represented by the following formula (2) from an epoxy compound having two or more units of the structure represented by the following formula (1); and (B) adding a mixed solvent of a protic polar solvent and an aprotic polar solvent to the reaction solution obtained after the step (A).

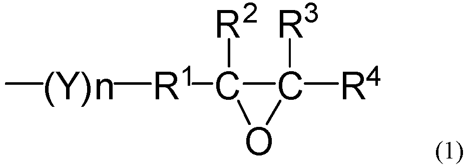

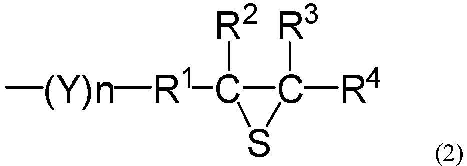

In formulae (1) and (2), R¹ represents a hydrocarbon group containing 0 to 10 carbon atoms, R², R³ and R⁴ each independently represent a hydrocarbon group containing 1 to 10 carbon atoms or a hydrogen atom, Y represents O, S, Se or Te, and n represents 0 or 1.

## Description

### Technical Field

The present invention relates to a production method for an episulfide compound, and particularly relates to a production method for an episulfide compound, wherein the production method is capable of suppressing the generation of viscous by-products.

### Background Art

Plastic lenses are light in weight and excellent in toughness, and are also easy to dye. Performance particularly required for plastic lenses includes low specific gravity, high transparency and low yellowness index, high refractive index and high Abbe's number (as optical performance), high heat resistance, high strength and so on. High refractive index allows a reduction in lens thickness, while high Abbe's number reduces chromatic aberration in lenses.

In recent years, there has been an ongoing development of optical material compositions each comprising a compound having episulfide groups, with the aim of achieving high refractive index and high Abbe's number (Patent Documents 1 and 2).

However, the production of a compound having episulfide groups has involved a problem in that with increase in the number of episulfide groups, viscous by-products will be deposited to cause pipe blockages. Upon occurrence of pipe blockages, mass production will be made difficult.

### Prior Art Documents

### Patent Documents

Patent Document 1: WO2018/150951
Patent Document 2: WO2020/031815

### Summary of the Invention

### Problem to be Solved by the Invention

The problem of the present invention is to provide a production method for an episulfide compound, wherein the production method is capable of suppressing the generation of viscous by-products.

### Means to Solve the Problem

Under these circumstances, as a result of repeating extensive and intensive efforts, the inventors of the present invention have found that the generation of viscous by-products can be suppressed when a mixed solvent of a protic polar solvent and an aprotic polar solvent is used as a reaction quencher. This finding led to the completion of the present invention.

Namely, the present invention is as follows.
<1> A production method for an episulfide compound, which comprises the steps of:
   (A) producing an episulfide compound having two or more units of the structure represented by the following formula (2) from an epoxy compound having two or more units of the structure represented by the following formula (1); and
   (B) adding a mixed solvent of a protic polar solvent and an aprotic polar solvent to the reaction solution obtained after the step (A) 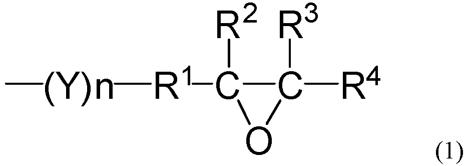
      wherein R¹ represents a hydrocarbon group containing 0 to 10 carbon atoms, R², R³ and R⁴ each independently represent a hydrocarbon group containing 1 to 10 carbon atoms or a hydrogen atom, Y represents O, S, Se or Te, and n represents 0 or 1, 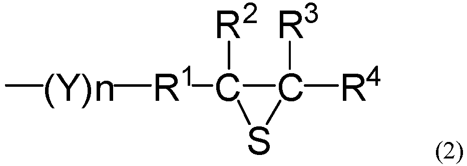
      wherein R¹ represents a hydrocarbon group containing 0 to 10 carbon atoms, R², R³ and R⁴ each independently represent a hydrocarbon group containing 1 to 10 carbon atoms or a hydrogen atom, Y represents O, S, Se or Te, and n represents 0 or 1.
<2> The production method for an episulfide compound according to <1> above, wherein the protic polar solvent is at least one selected from the group consisting of water, an aqueous acidic solution and an alcohol.
<3> The production method for an episulfide compound according to <2> above, wherein the aqueous acidic solution is at least one selected from the group consisting of an aqueous sulfuric acid solution, an aqueous hydrochloric acid solution, an aqueous nitric acid solution, an aqueous formic acid solution, an aqueous perchloric acid solution, an aqueous boric acid solution, an aqueous hydrofluoric acid solution, an aqueous acetic acid solution, an aqueous phosphoric acid solution, an aqueous hydroiodic acid solution, an aqueous hydrobromic acid solution, an aqueous phosphonic acid solution, an aqueous phosphinic acid solution, an aqueous diphosphoric acid solution, an aqueous metaphosphoric acid solution, an aqueous sulfurous acid solution, an aqueous chlorosulfonic acid solution, an aqueous carbonic acid solution, an aqueous propionic acid solution and an aqueous oxalic acid solution.
<4> The production method for an episulfide compound according to <2> above, wherein the alcohol is at least one selected from the group consisting of allyl alcohol, 2-aminoethanol, benzyl alcohol, 1-butanol, 2-butanol, cyclohexanol, methanol, ethanol, 2-chloroethanol, 2-butoxyethanol, 2-ethoxyethanol, 2-ethylhexanol, furfuryl alcohol, 1-hexanol, 2-methoxyethanol, 2-methyl-2-butanol, isopentyl alcohol, 1-octanol, 2-octanol, 1-pentanol, 3-pentanol, phenol and 1-propanol.
<5> The production method for an episulfide compound according to any one of <1> to <4> above, wherein the aprotic polar solvent is at least one selected from the group consisting of a sulfoxide, a nitrile, an amide, a ketone, an ester and an ether.
<6> The production method for an episulfide compound according to <5> above, wherein the sulfoxide is at least one selected from the group consisting of dimethyl sulfoxide, phenyl vinyl sulfoxide, 2-bromophenyl methyl sulfoxide, phenyl trifluoromethyl sulfoxide and 1-isothiocyanato-4-(methylsulfinyl)butane.
<7> The production method for an episulfide compound according to <5> above, wherein the nitrile is at least one selected from the group consisting of acetonitrile, thiophene-3-acetonitrile, thiophene-2-acetonitrile, 3-(methylamino)propionitrile, (dimethylamino)acetonitrile, methoxyacetonitrile, phenylacetonitrile, methylaminoacetonitrile, diethylaminoacetonitrile, 2-phenylpropionitrile and thiophene-2-acetonitrile.
<8> The production method for an episulfide compound according to <5> above, wherein the amide is at least one selected from the group consisting of dimethylformamide, dimethylacetamide, N-methylpropionamide, N,N-dimethylformamide dineopentylacetal, N-methylmethacrylamide, N-(isobutoxymethyl)acrylamide, N-(butoxymethyl)acrylamide, N,N-diethyl-M-toluamide, N,O-bis(trimethylsilyl)acetamide, 2,2'-(methylimino)bis(N,N-di-N-octylacetamide) and N-vinylformamide.
<9> The production method for an episulfide compound according to <5> above, wherein the ketone is at least one selected from the group consisting of acetone, methyl ethyl ketone, methyl isobutyl ketone, diisopropyl ketone, butyl isopropyl ketone, SEC-butyl methyl ketone, isobutyl isopropyl ketone, isopropyl methyl ketone, phenyl 1-propenyl ketone, dicyclohexyl ketone, isobutyl phenyl ketone, methyl phenyl ketone, methyl vinyl ketone, 3-octanone, 3-nonanone, 3-methyl-1-phenyl-2-butanone, 5-methyl-2-hexanone and 1-phenyl-2-butanone.
<10> The production method for an episulfide compound according to <5> above, wherein the ester is at least one selected from the group consisting of ethyl acetate, butyl acetate, normal propyl acetate, isobutyl acetate, pentyl acetate, isopentyl acetate, octyl acetate, methyl butyrate, ethyl butyrate, pentyl butyrate, methyl salicylate, ethyl formate, ethyl propionate, ethyl caproate and pentyl valerate.
<11> The production method for an episulfide compound according to <5> above, wherein the ether is at least one selected from the group consisting of tetrahydrofuran, 1,4-dioxane, anisole, diethyl ether, ethyl methyl ether, N-propyl ether, dibutyl ether, diphenyl ether, diethylene glycol monobutyl ether, ethylene glycol, diethylene glycol, diethylene glycol monoethyl ether, diethylene glycol monomethyl ether, ethylene oxide, benzofuran, dibenzofuran and crown ether.
<12> The production method for an episulfide compound according to any one of <1> to <11> above, wherein the step (A) is a step where at least acetic anhydride and thiourea are reacted with the epoxy compound having two or more units of the structure represented by formula (1) to thereby produce the episulfide compound having two or more units of the structure represented by formula (2).
<13> The production method for an episulfide compound according to any one of <1> to <12> above, which further comprises a step where at least epichlorohydrin is reacted with a compound having two or more mercapto groups to thereby produce the epoxy compound having two or more units of the structure represented by formula (1).

### Effects of the Invention

The present invention enables the provision of a production method for an episulfide compound, wherein the production method is capable of suppressing the generation of viscous by-products.

### Description of Embodiments

The present invention will be described in more detail by way of production examples and working examples illustrated below, although the present invention is not limited to these production examples and working examples illustrated below and can be implemented with modifications as appropriate without significantly departing from the spirit of the present invention.

The present invention is directed to a production method for an episulfide compound, which comprises the steps of: (A) producing an episulfide compound having two or more units of the structure represented by the following formula (2) from an epoxy compound having two or more units of the structure represented by the following formula (1); and (B) adding a mixed solvent of a protic polar solvent and an aprotic polar solvent to the reaction solution obtained after the step (A).

### <Step (A)>

In one embodiment of the present invention, an epoxy compound having two or more (preferably 2 to 10, more preferably 2 to 4) units of the structure represented by the following formula (1) may be reacted with a thiating agent (e.g., thiourea) to thereby produce an episulfide compound having two or more (preferably 2 to 10, more preferably 2 to 4) units of the structure represented by the following formula (2).

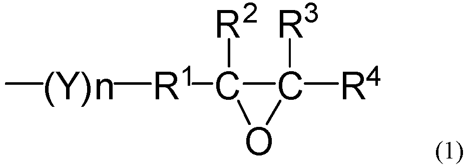

In formula (1), R¹ represents a hydrocarbon group containing 0 to 10 carbon atoms, preferably represents a hydrocarbon group containing 0 to 2 carbon atoms, and more preferably represents a methylene group.

R², R³ and R⁴ each independently represent a hydrocarbon group containing 1 to 10 carbon atoms or a hydrogen atom, preferably represent a hydrocarbon group containing 1 to 2 carbon atoms or a hydrogen atom, and more preferably represent a hydrogen atom.

Y represents O, S, Se or Te, preferably represents O or S, and more preferably represents S.

n represents 0 or 1, and preferably represents 1.

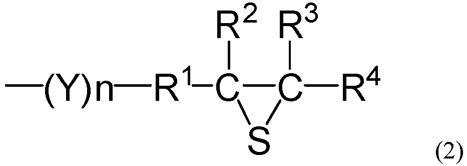

In formula (2), R¹ to R⁴, Y and n are as defined above for formula (1).

In the present invention, the above episulfide compound having two or more units of the structure represented by formula (2) is not limited in any way. However, specific examples include 1,3,5-tris(β-epithiopropyl)benzene, tetrakis(β-epithiopropylthiomethyl)methane, bis(β-epithiopropyl)sulfide, 1,5-bis(2,3-epithiopropoxy)naphthalene and so on.

In the step of obtaining the above episulfide compound having two or more units of the structure represented by formula (2) through the above reaction, a thiating agent (e.g., thiourea) may be used in a stoichiometric amount (i.e., number of moles) corresponding to epoxy groups in the above epoxy compound having two or more units of the structure represented by formula (1). If emphasis is laid on the reaction rate and the purity, the thiating agent may be used in a stoichiometric amount to a 2.5-fold stoichiometric amount, preferably in a 1.3-fold stoichiometric amount to a 2.0-fold stoichiometric amount, and more preferably in a 1.5-fold stoichiometric amount to a 2.0-fold stoichiometric amount. Examples of a polar organic solvent in which thiourea is soluble include alcohols (e.g., methanol, ethanol), ethers (e.g., diethyl ether, tetrahydrofuran, dioxane) and hydroxy ethers (e.g., methyl cellosolve, ethyl cellosolve, butyl cellosolve), with alcohols being preferred, and methanol being most preferred.

Examples of a nonpolar organic solvent in which the above epoxy compound having two or more units of the structure represented by formula (1) is soluble include aliphatic hydrocarbons (e.g., pentane, hexane, heptane), aromatic hydrocarbons (e.g., benzene, toluene) and halogenated hydrocarbons (e.g., dichloromethane, chloroform, chlorobenzene), with aromatic hydrocarbons being preferred, and toluene being most preferred. As to the solvent ratio used, preferred is the polar organic solvent/nonpolar organic solvent ratio = 0.1 to 10.0 by volume, and more preferred is the polar organic solvent/nonpolar organic solvent ratio = 0.2 to 5.0 by volume. If the ratio by volume is less than 0.1, thiourea is insufficiently soluble, so that the reaction does not proceed sufficiently. If the ratio by volume exceeds 10.0, polymer generation becomes noticeable. The reaction temperature is preferably 10°C to 30°C. If the reaction temperature is less than 10°C, the reaction rate is reduced and thiourea is insufficiently soluble, so that the reaction does not proceed sufficiently. If the reaction temperature exceeds 30°C, polymer generation becomes noticeable.

During the reaction, it is preferable to add an acid or an acid anhydride. Preferred for this purpose are acetic acid, propionic acid, butyric acid, succinic acid, maleic acid, benzoic acid, phthalic acid, pyromellitic acid, trimellitic acid, trifluoroacetic acid and acid anhydrides thereof, and most preferred are acetic acid and an acid anhydride thereof. The amount to be added is preferably in the range of 0.001% by mass to 10% by mass relative to the total mass of the reaction solution, and more preferably in the range of 0.01% by mass to 5% by mass relative to the total mass of the reaction solution. If the amount to be added is less than 0.001% by mass, polymer generation becomes noticeable, so that the reaction yield is reduced. If the amount to be added exceeds 10% by mass, the yield is significantly reduced.

In one embodiment of the present invention, the step (A) is particularly preferably a step where at least acetic anhydride and thiourea are reacted with the epoxy compound having two or more units of the structure represented by formula (1) to thereby produce the episulfide compound having two or more units of the structure represented by formula (2).

Moreover, in one embodiment of the present invention, the production method preferably further comprises a step where at least epichlorohydrin is reacted with a compound having two or more (preferably 2 to 10, more preferably 2 to 4) mercapto groups to thereby produce the epoxy compound having two or more (preferably 2 to 10, more preferably 2 to 4) units of the structure represented by formula (1).

### <Step (B)>

In the present invention, the step (B) is configured to add a mixed solvent of a protic polar solvent and an aprotic polar solvent to the reaction solution obtained after the step (A).

There is no particular limitation on how to add the mixed solvent to the reaction solution obtained after the step (A), and any technique commonly used in the art may be used for this purpose.

To confirm the completion of the above step (A), for example, HPLC analysis may be conducted to confirm that the reaction no longer proceeds, without being limited thereto.

Upon addition of a mixed solvent of a protic polar solvent and an aprotic polar solvent to the reaction solution obtained after the step (A), the deposition of viscous by-products can be suppressed. In particular, during the production of a bifunctional or higher functional episulfide compound, viscous by-products are highly likely to be deposited. Thus, the present invention is particularly suitable for the production of a bifunctional or higher functional episulfide compound.

According to the present invention, it is possible to solve the problem that with increase in the number of episulfide groups in a source material, viscous by-products will be deposited to cause pipe blockages. Thus, the present invention is preferred in terms of mass production.

In one embodiment of the present invention, the components in the mixed solvent used as a reaction quencher are preferably in the range of 1:10 to 10:1 protic polar solvent:aprotic polar solvent ratio by mass, and more preferably in the range of 1:1 to 1:4 protic polar solvent: aprotic polar solvent ratio by mass. Such a range allows effective suppression of the deposition of viscous by-products.

In one embodiment of the present invention, relative to the above epoxy compound having two or more units of the structure represented by formula (1), the mixed solvent used as a reaction quencher is preferably in the range of 1:1 to 1:60 epoxy compound:mixed solvent ratio by mass, and more preferably in the range of 1:1 to 1:30 epoxy compound:mixed solvent ratio by mass. Such a range allows effective suppression of the deposition of viscous by-products and is also advantageous in terms of yield.

In the present invention, the protic polar solvent used in the step (B) is not limited in any way, but it is preferably at least one selected from the group consisting of water, an aqueous acidic solution and an alcohol. Among them, an aqueous acidic solution is more preferred.

The above aqueous acidic solution is not limited in any way, but it is preferably at least one selected from the group consisting of an aqueous sulfuric acid solution, an aqueous hydrochloric acid solution, an aqueous nitric acid solution, an aqueous formic acid solution, an aqueous perchloric acid solution, an aqueous boric acid solution, an aqueous hydrofluoric acid solution, an aqueous acetic acid solution, an aqueous phosphoric acid solution, an aqueous hydroiodic acid solution, an aqueous hydrobromic acid solution, an aqueous phosphonic acid solution, an aqueous phosphinic acid solution, an aqueous diphosphoric acid solution, an aqueous metaphosphoric acid solution, an aqueous sulfurous acid solution, an aqueous chlorosulfonic acid solution, an aqueous carbonic acid solution, an aqueous propionic acid solution and an aqueous oxalic acid solution. Among them, an aqueous sulfuric acid solution, an aqueous hydrochloric acid solution and an aqueous formic acid solution are more preferred.

The acid concentration in the above aqueous acidic solution may be adjusted as appropriate depending on the type of acid, but it is preferably 0.1% to 30% by mass, and more preferably 5% to 20% by mass.

The above alcohol is not limited in any way, but it is preferably at least one selected from the group consisting of allyl alcohol, 2-aminoethanol, benzyl alcohol, 1-butanol, 2-butanol, cyclohexanol, methanol, ethanol, 2-propanol, 2-chloroethanol, 2-butoxyethanol, 2-ethoxyethanol, 2-ethylhexanol, furfuryl alcohol, 1-hexanol, 2-methoxyethanol, 2-methyl-2-butanol, isopentyl alcohol, 1-octanol, 2-octanol, 1-pentanol, 3-pentanol, phenol and 1-propanol. Among them, methanol, ethanol and 2-propanol are more preferred.

In the present invention, the aprotic polar solvent used in the step (B) is not limited in any way, but it is preferably at least one selected from the group consisting of a sulfoxide, a nitrile, an amide, a ketone, an ester and an ether. Among them, a sulfoxide and an amide are more preferred.

The above sulfoxide is not limited in any way, but it is preferably at least one selected from the group consisting of dimethyl sulfoxide, phenyl vinyl sulfoxide, 2-bromophenyl methyl sulfoxide, phenyl trifluoromethyl sulfoxide and 1-isothiocyanato-4-(methylsulfinyl)butane. Among them, dimethyl sulfoxide and phenyl vinyl sulfoxide are more preferred.

The above nitrile is not limited in any way, but it is preferably at least one selected from the group consisting of acetonitrile, thiophene-3-acetonitrile, thiophene-2-acetonitrile, 3-(methylamino)propionitrile, (dimethylamino)acetonitrile, methoxyacetonitrile, phenylacetonitrile, methylaminoacetonitrile, diethylaminoacetonitrile, 2-phenylpropionitrile and thiophene-2-acetonitrile. Among them, acetonitrile and methoxyacetonitrile are more preferred.

The above amide is not limited in any way, but it is preferably at least one selected from the group consisting of dimethylformamide, dimethylacetamide, N-methylpropionamide, N,N-dimethylformamide dineopentylacetal, N-methylmethacrylamide, N-(isobutoxymethyl)acrylamide, N-(butoxymethyl)acrylamide, N,N-diethyl-M-toluamide, N,O-bis(trimethylsilyl)acetamide, 2,2'-(methylimino)bis(N,N-di-N-octylacetamide) and N-vinylformamide. Among them, dimethylformamide and dimethylacetamide are more preferred.

The above ketone is not limited in any way, but it is preferably at least one selected from the group consisting of acetone, methyl ethyl ketone, methyl isobutyl ketone, diisopropyl ketone, butyl isopropyl ketone, SEC-butyl methyl ketone, isobutyl isopropyl ketone, isopropyl methyl ketone, phenyl 1-propenyl ketone, dicyclohexyl ketone, isobutyl phenyl ketone, methyl phenyl ketone, methyl vinyl ketone, 3-octanone, 3-nonanone, 3-methyl-1-phenyl-2-butanone, 5-methyl-2-hexanone and 1-phenyl-2-butanone. Among them, acetone and methyl ethyl ketone are more preferred.

The above ester is not limited in any way, but it is preferably at least one selected from the group consisting of ethyl acetate, butyl acetate, normal propyl acetate, isobutyl acetate, pentyl acetate, isopentyl acetate, octyl acetate, methyl butyrate, ethyl butyrate, pentyl butyrate, methyl salicylate, ethyl formate, ethyl propionate, ethyl caproate and pentyl valerate. Among them, ethyl acetate and butyl acetate are more preferred.

The above ether is not limited in any way, but it is preferably at least one selected from the group consisting of tetrahydrofuran, 1,4-dioxane, anisole, diethyl ether, ethyl methyl ether, N-propyl ether, dibutyl ether, diphenyl ether, diethylene glycol monobutyl ether, ethylene glycol, diethylene glycol, diethylene glycol monoethyl ether, diethylene glycol monomethyl ether, ethylene oxide, benzofuran, dibenzofuran and crown ether. Among them, tetrahydrofuran and diethyl ether are more preferred.

### Examples

The details of the present invention will be described by way of the following examples and comparative examples, although the present invention is not limited to the following examples. It should be noted that viscous by-products and the yield were evaluated according the criteria shown below.

### <Viscous by-products>

A: visually undetectable
B: slightly deposited
C: deposited to cause funnel blockage
-: not evaluated because the yield of the desired product is 0%

### <Yield>

A: 50% or higher
B: less than 50%
C: the desired product cannot be obtained

### (Example 1)

### (1) Synthesis of episulfide compound material

A 2 L three-necked flask was equipped with a thermocouple, a bubbling tube and a dropping funnel. After the inside of the flask was purged with nitrogen, trimercaptobenzene (70.00 g) and toluene (303.38 g) were added and stirred at 5°C. Then, a mixed solution of 24% aqueous sodium hydroxide (3.34 g) and methanol (110.85 g) was added, and the temperature within the flask was maintained at 5°C. Subsequently, epichlorohydrin (115.19 g) was added dropwise, and the reaction was continued for 3 hours. Then, 24% aqueous sodium hydroxide (334.69 g) was added through the dropping funnel while maintaining the temperature within the flask at 15°C. After dropwise addition, the solution temperature was set to 15°C, and stirring was conducted for 24 hours. At 24 hours after addition of the aqueous sodium hydroxide, methanol (167.96 g) was added to the reaction solution, followed by stirring until the intermediate area (%) reached ≤1.0% as measured by HPLC analysis. Then, water (700.0 g) was added to quench the reaction. The reaction solution was transferred to a separatory funnel, and the organic layer was washed three times with water (700.0 g), followed by collecting the organic layer. The organic layer was concentrated by an evaporator and dried under vacuum to obtain an epoxy compound represented by the following formula (3) (hereinafter also referred to as "compound 3").

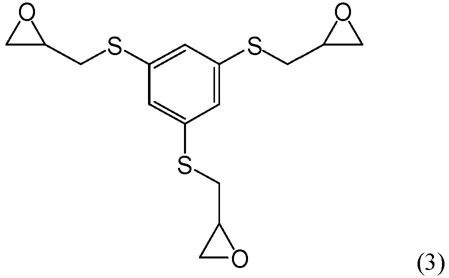

### (2) Synthesis of 1,3,5-tris(β-epithiopropyl)benzene

A 2 L recovery flask was charged with 20.00 g of the above epoxy compound represented by formula (3) and equipped with a bubbling tube. Then, toluene (86.68 g), methanol (79.18 g) and acetic anhydride (1.07 g) were added, and the solution temperature was set to 20°C, followed by stirring in an open system. Then, thiourea (20.00 g) was added, and stirring was conducted at 20°C for 26 hours. After confirming that the reaction no longer proceeded as measured by HPLC analysis, 600.00 g of a mixed solvent containing 20% sulfuric acid (200.00 g) as a protic polar solvent and dimethyl sulfoxide (DMSO) (400.00 g) as an aprotic polar solvent (20% sulfuric acid:DMSO = 1:2) was added as a reaction quencher to the reaction solution (compound 3:mixed solvent = 1:30), thereby quenching the reaction. The solution temperature was set to 20°C, and stirring was conducted for 1 hour. After stirring, the solution was transferred to a separatory funnel. In the recovery flask used as a reaction vessel, no viscous by-products were confirmed. The separatory funnel was allowed to stand for partition, and the lower layer was then discarded while the organic layer was collected. 20% Sulfuric acid (200.00 g) was added to the organic layer to conduct acid washing, followed by removal of the aqueous layer. Then, the same operation was repeated to wash the organic layer three times with water (200.00 g). The collected organic layer was concentrated and then dried at 40°C under vacuum to obtain an episulfide compound represented by the following formula (4), i.e., 1,3,5-tris(β-epithiopropyl)benzene as a yellow liquid in a yield of 50% to 60% with an HPLC purity (area %) of >98% and an HPLC purity (gravimetry) of >97%. In the reactor after the reaction, only white solids were deposited and no viscous by-products were confirmed. In addition, the vessel after the reaction was able to be easily washed with THF.

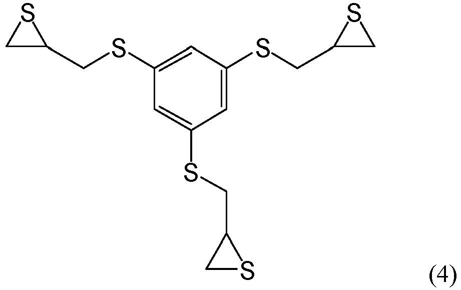

### (Example 2)

The same procedure as shown in Example 1 was repeated to produce an episulfide compound, except that Example 1 was modified such that the mass ratio of the components in the mixed solvent (20% sulfuric acid:DMSO) used as a reaction quencher was changed from 1:2 to 10:1. The degree of generation of viscous by-products in the flask after the reaction is shown in Table 1 below.

### (Example 3)

The same procedure as shown in Example 1 was repeated to produce an episulfide compound, except that Example 1 was modified such that the mass ratio of the components in the mixed solvent (20% sulfuric acid:DMSO) used as a reaction quencher was changed from 1:2 to 1:10. The degree of generation of viscous by-products in the flask after the reaction is shown in Table 1 below.

### (Example 4)

The same procedure as shown in Example 1 was repeated to produce an episulfide compound, except that Example 1 was modified such that the added ratio of compound 3 and the mixed solvent was changed from 1:30 to 1:1. The degree of generation of viscous by-products in the flask after the reaction is shown in Table 1 below.

### (Example 5)

The same procedure as shown in Example 1 was repeated to produce an episulfide compound, except that Example 1 was modified such that the added ratio of compound 3 and the mixed solvent was changed from 1:30 to 1:60. The degree of generation of viscous by-products in the flask after the reaction is shown in Table 1 below.

### (Example 6)

The same procedure as shown in Example 1 was repeated to produce an episulfide compound, except that Example 1 was modified such that 5% sulfuric acid was used as a protic polar solvent, instead of 20% sulfuric acid. The degree of generation of viscous by-products in the flask after the reaction is shown in Table 1 below.

### (Example 7)

The same procedure as shown in Example 1 was repeated to produce an episulfide compound, except that Example 1 was modified such that 30% sulfuric acid was used as a protic polar solvent, instead of 20% sulfuric acid. The degree of generation of viscous by-products in the flask after the reaction is shown in Table 1 below.

### (Example 8)

The same procedure as shown in Example 1 was repeated to produce an episulfide compound, except that Example 1 was modified such that N,N-dimethylformamide (DMF) was used as an aprotic polar solvent, instead of DMSO. The degree of generation of viscous by-products in the flask after the reaction is shown in Table 1 below.

### (Example 9)

A 2 L recovery flask was charged with a tetramercaptopentaerythritol-derived tetrafunctional epoxy compound represented by the following formula (5), instead of the above epoxy compound represented by formula (3), and was equipped with a bubbling tube. Then, toluene (86.68 g), methanol (79.18 g) and acetic anhydride (1.15 g) were added, and the solution temperature was set to 20°C, followed by stirring in an open system. Then, thiourea (21.51 g) was added, and stirring was conducted at 20°C for 24 hours. After confirming that the reaction no longer proceeded as measured by HPLC analysis, 600.00 g of a mixed solvent containing 20% sulfuric acid (200.00 g) as a protic polar solvent and DMSO (400.00 g) as an aprotic polar solvent (20% sulfuric acid:DMSO = 1:2) was added as a reaction quencher to the reaction solution (compound 3:mixed solvent = 1:30), thereby quenching the reaction. The solution temperature was set to 20°C, and stirring was conducted for 1 hour. After stirring, the solution was transferred to a separatory funnel. In the recovery flask used as a reaction vessel, no viscous by-products were confirmed. The separatory funnel was allowed to stand for partition, and the lower layer was then discarded while the organic layer was collected. 20% Sulfuric acid (200.00 g) was added to the organic layer to conduct acid washing, followed by removal of the aqueous layer. Then, the same operation was repeated to wash the organic layer three times with water (200.00 g). The collected organic layer was concentrated and then dried at 40°C under vacuum to obtain an episulfide compound represented by the following formula (6), i.e., tetrakis(β-epithiopropylthiomethyl)methane as a yellow liquid in a yield of 50% to 60% with an HPLC purity (area %) of >95% and an HPLC purity (gravimetry) of >96%. In the reactor after the reaction, only white solids were deposited and no viscous by-products were confirmed. In addition, the vessel after the reaction was able to be easily washed with THF.

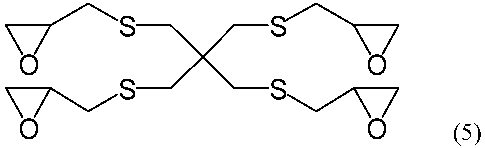

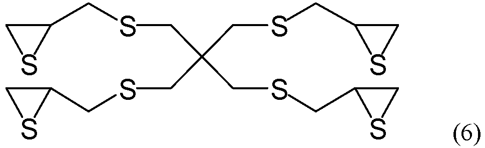

### (Comparative Example 1)

The same procedure as shown in Example 1 was repeated to produce an episulfide compound, except that Example 1 was modified such that the reaction quencher contained no aprotic polar solvent. The degree of generation of viscous by-products in the flask after the reaction is shown in Table 1 below.

### (Comparative Example 2)

The same procedure as shown in Example 1 was repeated to produce an episulfide compound, except that Example 1 was modified such that the reaction quencher contained no protic polar solvent. The degree of generation of viscous by-products in the flask after the reaction is shown in Table 1 below.

### (Comparative Example 3)

The same procedure as shown in Example 1 was repeated to produce an episulfide compound, except that Example 1 was modified such that toluene serving as a nonpolar solvent was used instead of DMSO serving as an aprotic polar solvent. The degree of generation of viscous by-products in the flask after the reaction is shown in Table 1 below.

Table 1 shows the results evaluated for the evaluation items of Examples 1 to 9 and Comparative Examples 1 to 3.

**[Table 1]**

| | | Protic polar solvent | Aprotic polar solvent | Nonpolar solvent | Protic polar solvent: aprotic polar solvent (mass ratio) | Compound 3: mixed solvent (mass ratio) | Viscous by-product | Yield |
|---|---|---|---|---|---|---|---|---|
| Example 1 | Trimercaptobenzene | 20% Sulfuric acid | DMSO | - | 1:2 | 1:30 | A | A |
| Example 2 | Trimercaptobenzene | 20% Sulfuric acid | DMSO | - | 10:1 | 1:30 | B | A |
| Example 3 | Trimercaptobenzene | 20% Sulfuric acid | DMSO | - | 1:10 | 1:30 | A | B |
| Example 4 | Trimercaptobenzene | 20% Sulfuric acid | DMSO | - | 1:2 | 1:1 | B | A |
| Example 5 | Trimercaptobenzene | 20% Sulfuric acid | DMSO | - | 1:2 | 1:60 | A | B |
| Example 6 | Trimercaptobenzene | 5% Sulfuric acid | DMSO | - | 1:2 | 1:30 | B | A |
| Example 7 | Trimercaptobenzene | 30% Sulfuric acid | DMSO | - | 1:2 | 1:30 | A | A |
| Example 8 | Trimercaptobenzene | 20% Sulfuric acid | DMF | - | 1:2 | 1:30 | A | B |
| Example 9 | Tetramercaptopentaerythritol | 20% Sulfuric acid | DMSO | - | 1:2 | 1:30 | A | A |
| Comparative Example 1 | Trimercaptobenzene | 20% Sulfuric acid | - | - | - (1:0) | 1:30 | C | A |
| Comparative Example 2 | Trimercaptobenzene | - | DMSO | - | - (0:1) | 1:30 | | C |
| Comparative Example 3 | Trimercaptobenzene | 20% Sulfuric acid | - | Toluene | (20% Sulfuric acid: toluene = 1:2) | 1:30 | C | B |

## Claims

1. A production method for an episulfide compound, which comprises the steps of:
(A) producing an episulfide compound having two or more units of the structure represented by the following formula (2) from an epoxy compound having two or more units of the structure represented by the following formula (1); and
(B) adding a mixed solvent of a protic polar solvent and an aprotic polar solvent to a reaction solution obtained after the step (A) 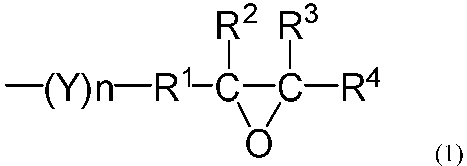
wherein R¹ represents a hydrocarbon group containing 0 to 10 carbon atoms, R², R³ and
R⁴ each independently represent a hydrocarbon group containing 1 to 10 carbon atoms or a hydrogen atom, Y represents O, S, Se or Te, and n represents 0 or 1, 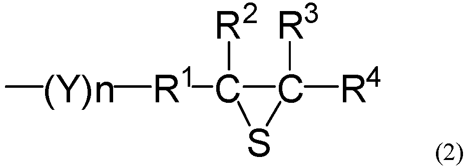
wherein R¹ represents a hydrocarbon group containing 0 to 10 carbon atoms, R², R³ and
R⁴ each independently represent a hydrocarbon group containing 1 to 10 carbon atoms or a hydrogen atom, Y represents O, S, Se or Te, and n represents 0 or 1.

2. The production method for an episulfide compound according to claim 1, wherein the protic polar solvent is at least one selected from the group consisting of water, an aqueous acidic solution and an alcohol.

3. The production method for an episulfide compound according to claim 2, wherein the aqueous acidic solution is at least one selected from the group consisting of an aqueous sulfuric acid solution, an aqueous hydrochloric acid solution, an aqueous nitric acid solution, an aqueous formic acid solution, an aqueous perchloric acid solution, an aqueous boric acid solution, an aqueous hydrofluoric acid solution, an aqueous acetic acid solution, an aqueous phosphoric acid solution, an aqueous hydroiodic acid solution, an aqueous hydrobromic acid solution, an aqueous phosphonic acid solution, an aqueous phosphinic acid solution, an aqueous diphosphoric acid solution, an aqueous metaphosphoric acid solution, an aqueous sulfurous acid solution, an aqueous chlorosulfonic acid solution, an aqueous carbonic acid solution, an aqueous propionic acid solution and an aqueous oxalic acid solution.

4. The production method for an episulfide compound according to claim 2, wherein the alcohol is at least one selected from the group consisting of allyl alcohol, 2-aminoethanol, benzyl alcohol, 1-butanol, 2-butanol, cyclohexanol, methanol, ethanol, 2-chloroethanol, 2-butoxyethanol, 2-ethoxyethanol, 2-ethylhexanol, furfuryl alcohol, 1-hexanol, 2-methoxyethanol, 2-methyl-2-butanol, isopentyl alcohol, 1-octanol, 2-octanol, 1-pentanol, 3-pentanol, phenol and 1-propanol.

5. The production method for an episulfide compound according to any one of claims 1 to 4, wherein the aprotic polar solvent is at least one selected from the group consisting of a sulfoxide, a nitrile, an amide, a ketone, an ester and an ether.

6. The production method for an episulfide compound according to claim 5, wherein the sulfoxide is at least one selected from the group consisting of dimethyl sulfoxide, phenyl vinyl sulfoxide, 2-bromophenyl methyl sulfoxide, phenyl trifluoromethyl sulfoxide and 1-isothiocyanato-4-(methylsulfinyl)butane.

7. The production method for an episulfide compound according to claim 5, wherein the nitrile is at least one selected from the group consisting of acetonitrile, thiophene-3-acetonitrile, thiophene-2-acetonitrile, 3-(methylamino)propionitrile, (dimethylamino)acetonitrile, methoxyacetonitrile, phenylacetonitrile, methylaminoacetonitrile, diethylaminoacetonitrile, 2-phenylpropionitrile and thiophene-2-acetonitrile.

8. The production method for an episulfide compound according to claim 5, wherein the amide is at least one selected from the group consisting of dimethylformamide, dimethylacetamide, N-methylpropionamide, N,N-dimethylformamide dineopentylacetal, N-methylmethacrylamide, N-(isobutoxymethyl)acrylamide, N-(butoxymethyl)acrylamide, N,N-diethyl-M-toluamide, N,O-bis(trimethylsilyl)acetamide, 2,2'-(methylimino)bis(N,N-di-N-octylacetamide) and N-vinylformamide.

9. The production method for an episulfide compound according to claim 5, wherein the ketone is at least one selected from the group consisting of acetone, methyl ethyl ketone, methyl isobutyl ketone, diisopropyl ketone, butyl isopropyl ketone, SEC-butyl methyl ketone, isobutyl isopropyl ketone, isopropyl methyl ketone, phenyl 1-propenyl ketone, dicyclohexyl ketone, isobutyl phenyl ketone, methyl phenyl ketone, methyl vinyl ketone, 3-octanone, 3-nonanone, 3-methyl-1-phenyl-2-butanone, 5-methyl-2-hexanone and 1-phenyl-2-butanone.

10. The production method for an episulfide compound according to claim 5, wherein the ester is at least one selected from the group consisting of ethyl acetate, butyl acetate, normal propyl acetate, isobutyl acetate, pentyl acetate, isopentyl acetate, octyl acetate, methyl butyrate, ethyl butyrate, pentyl butyrate, methyl salicylate, ethyl formate, ethyl propionate, ethyl caproate and pentyl valerate.

11. The production method for an episulfide compound according to claim 5, wherein the ether is at least one selected from the group consisting of tetrahydrofuran, 1,4-dioxane, anisole, diethyl ether, ethyl methyl ether, N-propyl ether, dibutyl ether, diphenyl ether, diethylene glycol monobutyl ether, ethylene glycol, diethylene glycol, diethylene glycol monoethyl ether, diethylene glycol monomethyl ether, ethylene oxide, benzofuran, dibenzofuran and crown ether.

12. The production method for an episulfide compound according to any one of claims 1 to 11, wherein the step (A) is a step where at least acetic anhydride and thiourea are reacted with the epoxy compound having two or more units of the structure represented by formula (1) to thereby produce the episulfide compound having two or more units of the structure represented by formula (2).

13. The production method for an episulfide compound according to any one of claims 1 to 12, which further comprises a step where at least epichlorohydrin is reacted with a compound having two or more mercapto groups to thereby produce the epoxy compound having two or more units of the structure represented by formula (1).
